# EUROPEAN PATENT APPLICATION

(11) **EP 1 013 274 A2**
(43) Date of publication of application: **28.06.2000**
(21) Application number: 00200560.1
(22) Date of filing: 02.05.1996
(51) Int. Cl.: A61K 31/43

(54) **Composition comprising amoxycillin and clavulanic acid**

(30) Priority: 03.05.1995 GB 9508989; 18.11.1995 GB 9523655
(62) Divisional of application: 96919679.9
(71) Applicant: SMITHKLINE BEECHAM PLC, Brentford, Middlesex TW8 9EP (GB); SMITHKLINE BEECHAM CORPORATION, Philadelphia Pennsylvania 19103 (US)
(72) Inventor: Bax, Richard Peregrine, Smithkline Beecham Pharm., Harlow, Essex, CM19 5AW (GB); Ramsey, Mike Gale, Smithkline Beecham Pharm., Bristol, TN 37620 (US)
(74) Representative: Connell, Anthony Christopher

(57) **Abstract**

Paediatric aqueous liquid suspension formulations comprising amoxycillin trihydrate and potassium clavulanate in a ratio of from 6:1 to 8:1 are provided for use in a BID dosage regimen for treating bacterial infection.

## Description

This invention relates to a method of treating paediatric bacterial infections using a combination of the antibiotic amoxycillin and the beta-lactamase inhibitor potassium clavulanate, to pharmaceutical formulations for use in such method and to methods for the manufacture of such formulations.

The combination of the antibiotic amoxycillin, as amoxycillin trihydrate, and potassium clavulanate, is a well known and widely used oral medicament for bacterial infections, marketed by SmithKline Beecham in many countries under the trade mark Augmentin.

Regulatory approval has been obtained, for instance in the UK and US, for tablets containing amoxycillin (250mg) and potassium clavulanate (125mg) (ratio 2:1), in a three times daily dosing schedule (tid), so that the daily dose of amoxycillin and potassium clavulanate is 750mg and 375mg respectively (the weights being expressed as the free parent acids amoxycillin and clavulanic acid, this manner of expression being used throughout). In severe infections, the ratio is changed to 4:1, so that the daily doses of amoxycillin and potassium clavulanate are 1500mg and 375mg respectively. In other countries such as Italy and Spain, tablets containing amoxycillin (875mg) and potassium clavulanate (125mg) (ratio 7:1) are approved for twice daily dosing (bid). In France, sachets comprising amoxycillin (1000mg) and potassium clavulanate (125mg) (ratio 8:1) are marketed, for reconstitution with water prior to use, as an individual dosage for adults.

For children, it is preferred to provide the combination as a powder which is reconstituted prior to use as a liquid aqueous suspension. The usual recommended daily dosage is 20/5mg/kg/day (UK and USA) or 30/7.5mg/kg/day (Continental Europe) amoxycillin/potassium clavulanate (ratio 4:1), in divided doses every eight hours. For more severe infections such as otitis media, sinusitis and lower respiraotory tract infections, the recommended dose is 40/10mg/kg/day (UK and USA) or 60/15mg/kg/day (Continental Europe), in three divided doses. For convenience, the powders are provided in amounts which are made up into a range of volumes of suspension so that a small volume, typically about 5ml, contains a unit dosage. In other countries such as Italy, approval has also been given for using the higher strength paediatric formulation on a twice a day (bid) dosing schedule.

Although it is recognised to be more convenient to be able to recommend a bid dosing schedule for paediatric formulations, to avoid having to give medicine during the middle of the day when the child may be at school, not all drug substances have pharmocokinetics which are compatible with such a regimen.

In addition, gastric intolerance, manifested in symptons such as loose stools, is perceived in some countries to be a side effect associated with the use of amoxycillin/potassium clavulanate in the present tid dosage regimes. Accordingly, any measures, such as revised dosage regimes, which can mitigate this would be advantageous.

The present invention therefore provides a method of treating bacterial infections in paediatric patients which method comprises administering to a patient in need thereof, preferably as a liquid aqueous suspension, amoxycillin trihydrate and potassium clavulanate in combination, in a weight ratio of between 6:1 and 8;1, preferably about 6.5:1 to 7.5:1, more preferably about 7:1 (the weights being expressed as the free parent acids amoxycillin and clavulanic acid), such administration being twice daily (bid) and at a dosage of between 15 and 80mg/kg/day, preferably 20 and 75mg/kg/day, more preferably 20 and 70mg/kg/day, suitably 40 and 70mg/kg/day, of amoxycillin and *pro rata* amounts of clavulanic acid.

The term 'paediatric' as used herein means children in the age range from 0 to about 12 years.

The method is suitable for all infections for which the combination of amoxycillin trihydrate and potassium clavulanate is normally prescribed, for instance infections of the upper and lower respiratory tract, urinary tract, skin and skin structures, for example *otitis media.*

Suitabably the twice daily (bid) administration is at 12 hour intervals, although a greater or lesser interval between administrations may be used.

Representative daily amounts of amoxycillin/potassium clavulanate include 25/3.6, 35/5, 45/6.4 and 70/10±10%mg/kg/day,

In a further aspect, the present invention provides for pharmaceutical compositions for use in the aforementioned method of treatment, these compositions being distinguishable over previously available compositions on account of the different ratio of amoxycillin trihydrate:potassium clavulanate used and the differing amounts of amoxycillin trihydrate and potassium clavulanate per unit dose, to achieve the higher daily dosage regime.

The invention therefore provides a pharmaceutical formulation suitable for paediatric oral bid administration, comprising amoxycillin trihydrate and potassium clavulanate in combination, in a weight ratio of between 6:1 and 8:1, preferably about 6.5:1 to 7.5:1, more preferably about 7:1 (the weights being expressed as the free parent acids amoxycillin and clavulanic acid), and in quantities such as to provide between between 15 and 80mg/kg/day, preferably 20 and 75mg/kg/day, more preferably 20 and 70mg/kg/day. suitably 40 and 70mg/kg/day, of amoxycillin and *pro rata* amounts of clavulanic acid.

Formulations according to the present invention are preferably provided in the form of a dry powder or granule formulation for reconstitution into an aqueous suspension with water or other suitable aqueous media, shortly before administration. The present invention covers such dry powder and granule formulations as well as liquid aqueous preparations.

Such dry formulations may be provided in a substantially air-tight container such as a bottle or sachet, and this container may suitably include a desiccant to protect the potassium clavulanate from degradation by atmospheric water vapour. Potassium clavulanate is highly moisture sensitive, and the formulations of this invention should be made under conditions of relative humidty (RH) as low as possible, preferably 30% RH or less.

The formulation of this invention is provided for bid administration, which ideally may comprise two administrations at 12 hour dosage intervals, although a greater or lesser interval between administrations may be used.

Suitably, formulations according to the present invention are provided in amounts such that the liquid aqueous suspension contains in a convenient volume, typically within the range 2 to 10ml. preferably about 5ml, of suspension, a unit dosage of amoxycillin and potassium clavulanate. The volume may be measured by any conventional measuring device such as a spoon, syringe or graduated measuring cup. Unit dosages typically lie within the range 50 to 800mg of amoxycillin plus *pro rata* amounts of potassium clavulanate. It will be appreciated that the appropriate unit dosage will be at the discretion of the physician and will depend *inter alia* upon the age and weight of the patient and the nature and severity of the infection to be treated.

It is found to be convenient to provide paediatric formulations in a lower (for mild-moderate infections) and a higher strength (for severe infections). The suitable formulations, when made up as aqueous liquid suspensions, will contain from 100 to 400mg, or 200 to 800mg amoxycillin per 5ml of suspension plus *pro rata* amounts of potassium clavulanate. Representative examples include:

| | |
|---|---|
| amoxycillin | potassium clavulanate |
| 200 | 28.5/5ml suspension |
| 400 | 57; |

within a tolerance of ±10%.

Accordingly, in a further aspect, the present invention provides a pharmaceutical formulation adapted for reconstitution as a liquid aqueous suspension comprising amoxycillin trihydrate and potassium clavulanate and which, when reconsituted, comprises amoxycillin in an amount 200±10% and clavulanic acid in an amount 28.5±10% or amoxycillin in an amount 400±10% and clavulanic acid in an amount 57±10% mg/5ml of liquid aqueous suspension.

In a representative example, for a unit dosages of 5ml, the suspension is made up to a total volume of 100ml. It will however be appreciated that a range of total volumes may be used, to adjust the amount in a unit dose to an amount appropriate for the individual patient. It will be appreciated that it may also be convenient to provide a higher strength formulation to a patient with a larger body weight, so that the unit volume is kept reasonable.

The formulation of this invention will normally comprise, in addition to its active materials amoxycillin trihydrate and potassium clavulanate, excipients which are standard in the field of paediatric pharmaceutical oral suspensions. These will be used in generally standard proportions, and at generally standard particle sizes and grades etc.. Such excipients may comprise suspending aids, glidants (to aid filling), diluents, bulking agent, flavours, sweeteners, stabilisers, and, in the case of dry formulations for make-up to an aqueous suspension, an edible desiccant to assist preservation of the potassium clavulanate against hydrolysis by atmospheric moisture on storage. Potassium clavulanate is normally supplied in admixture with silicon dioxide as diluent. Suitable excipients for use include xanthan gum (suspension aid), colloidal silica (glidant), succinic acid (stabiliser), aspartame (sweetener), hydroxypropylmethylcellulose (suspension aid) and silicon dioxide (desiccant, diluent for potassium clavulanate and bulking agent. Flavours may comprise common flavours such as orange, banana, raspberry and golden syrup, or mixtures thereof, to suit local requirements.

Mannitol is often used in pharmaceutical formulations as an excipient. It is however recognised to have, at least at certain levels, a diuretic effect. It has found to be advantageous to avoid the use of excessive amounts of mannitol in formulations comprising amoxycillin/potassium clavulanate, as it is thought that this may be associated with reduced levels of gastric irritancy. Accordingly, the present invention provides for a pharmaceutical formulation as hereinbefore defined and which is substantially mannitol-free.

Generally the proportion of active materials amoxycillin trihydrate and potassium clavulanate in a dry formulation for make-up with aqueous media into a suspension formulation of the invention may be around 35-60, e.g. 35-50 wt%.

The formulation of the invention may be manufactured using techniques which are generally conventional in the field of manufacture of paediatric suspension formulations and manufacture of dry formulations for reconstitution into such suspensions. For example a suitable technique is that of mixing dry powdered or granulated ingredients for loading into a suitable container.

The present invention therefore provides a process for manufacture of a formulation as described above.

The invention also provides for the use of amoxycillin trihydrate and potassium clavulanate as described above in the manufacture of a medicament which is provided for paediatric oral BID administration for use in the treatment of bacterial infections.

The invention will now be described by way of non-limiting example only.

### Example 1

Two formulations of this invention having a composition as listed below were prepared using conventional techniques as dry powder mixtures. The proportions of ingredients are expressed as mg/5ml dose of reconstituted aqueous suspension:

| **Ingredient** | **mg/5ml** | **mg/5ml** |
|---|---|---|
| amoxycillin trihydrate* | 408.0 | 204.0 |
| potassium clavulanate* | 61.56 | 30.78 |
| xanthan gum | 12.5 | 12.5 |
| colloidal silica | 25.0 | 25.0 |
| succinnic acid | 0.84 | 0.84 |
| orange flavour | 26.25 | 26.25 |
| golden syrup flavour | 23.75 | 23.75 |
| aspartame | 12.50 | 12.50 |
| hydroxypropylmethylcellulose | 79.65 | 79.65 |
| silicon dioxide | to 885.5 | to 537.5 |

| | | |
|---|---|---|
| * expressed as free acid equivalent. | | |

The above two formulations were manufactured in 100 kg batches.

### Clinical Trial - A

In a multicentre randomized trial, the safety and efficacy in the treatment of acute Otitis Media in children of formulations according to the present invention and comprising amoxycillin/potassium clavulanate in a ratio of 7:1, at a level of 45/6.4 mg/kg/day (ratio 7:1) and in divided doses q12h (BID) were compared with a currently approved US formulation comprising amoxycillin/potassium clavulanate in a ratio 4:1 administered at a level of 40/10mg/kg/day amoxycillin/potassium clavulanate acid in divided doses q 8h (TID). 287 children received the BID regimen for 10 days and 288 children received the TID regimen for 10 days. The formulations according to the present invention were mannitol-free whilst the currently approved US formulation contained mannitol.

The BID regimen was shown to be as safe as the TID regimen. Standard diary cards were used to assess the incidence of incidence of protocol-defined diarrhoea (i.e. 3 or more watery stools in one day, or 2 watery stools per day for two consecutive days. This was found to be significantly lower for the BID regimen (7.9%) when compared with the TID regimen (22.2%) [95% CI: (-20.5%, -8.1%)]. Similar trends were observed for withdrawals due to diarrhoea (2.8% and 7.6% respectively; p = 0.009), confirming improved patient tolerance compared with the current regimen.

The per protocol clinical success rates at the end of therapy (days 12 to 14) were equivalent for the BID regimen (86.5%) and the TID regimen (78.88%). Similar trends in efficacy were noted at follow-up (days 32 to 38).

### Formulations used

| **Ingredient** | **bid** | **bid** | **tid** | **tid** |
|---|---|---|---|---|
| | **mg/5ml** | **mg/5ml** | **mg/5ml** | **mg/5ml** |
| amoxycillin trihydrate* | 408.0 | 204.0 | 130.00 | 260.00 |
| potassium clavulanate* | 61.56 | 30.78 | 35.00 | 70.00 |
| xanthan gum | 12.5 | 12.5 | 15.00 | 15.00 |
| sodium saccharin | | | 4.00 | 4.00 |
| colloidal silica | 25.0 | 25.0 | 25.00 | 25.00 |
| succinnic acid | 0.84 | 0.84 | 0.85 | 0.85 |
| banana flavour | | | 20.00 | |
| orange flavour | 26.25 | 26.25 | | 23.00 |
| golden syrup flavour | 23.75 | 23.75 | | |
| aspartame | 12.50 | 12.50 | | |
| hydroxypropylmethylcellulose | 79.65 | 79.65 | | |
| silicon dioxide | to 885.5 | to 537.5 | 82.30 | 39.70 |
| mannitol | | | to 900.00 | to 1200.00 |

| | | | | |
|---|---|---|---|---|
| * expressed as free acid equivalent. | | | | |

### Clinical trial - B

In a multicentre randomized trial, the safety and efficacy in the treatment of acute Otitis Media in children of formulations according to the present invention and comprising amoxycillin/potassium clavulanate in a ratio of 7:1, at a level of 70/10mg/kg/day (ratio 7:1) and in two divided doses (BID) were compared with a currently approved European formulation comprising amoxycillin/potassium clavulanate in a ratio 4:1 administered at a level of 60/15mg/kg/day amoxycillin/potassium clavulanate acid in three divided doses (TID). Children aged from 2-12 years were randomised to 10 days treatment, with 231 children receiving the BID regimen and 232 children receiving the TID regimen.

The BID regimen was shown to be as safe as the TID regimen. Standard diary cards were used to assess the incidence of protocol-defined diarrhoea (i.e. 3 or more watery stools in one day, or 2 watery stools per day for two consecutive days). The overall incidence was found to be low, with a lower incidence in the BID group (6.7%) than in the TID group (10.3%) although this was not statistically significant [difference - 3.6%; 95% CI (-8.72%, 1.58%)].

Clinical success rates at the end of therapy were 91.8% for the BID regimen and 90.5% for the TID regimen [difference 1.3%,: 95% CI (-3.92%, 6.43%)] and at follow-up were 80.1% for the BID group and 77.6% for the TID group [difference 2.5%; 95% CI (-4.94%, 9.94%)].

More patients in the BID group (81.3%) than in the TID group (72.8%) had at least 80% compliance over a 7-10 day treatment period [difference 10.3%; 95% CI (2.78%. 17.76%)].

### Formulations used

| **Ingredient** | **bid formulation** | **tid formulation** |
|---|---|---|
| | **mg/5ml** | **mg/5ml** |
| amoxycillin trihydrate* | 400.0 | 250.00 |
| potassium clavulanate* | 59.85 | 65.63 |
| xanthan gum | 12.50 | 12.50 |
| colloidal silica | 25.00 | 25.00 |
| succinnic acid | 0.84 | 0.84 |
| orange flavour | 26.25 | 26.25 |
| raspberry flavour | | 22.50 |
| golden syrup flavour | 23.75 | 23.75 |
| aspartame | 12.50 | 12.50 |
| hydroxypropylmethylcellulose | 79.65 | 150.00 |
| silicon dioxide | to 885.5 | 125.00 |

| | | |
|---|---|---|
| * expressed as free acid equivalent | | |

## Claims

1. A multiple dosage pharmaceutical formulation in the form of a dry powder or a granule formulation for reconstitution into a suspension with water or other suitable aqueous media to form a suspension, comprising amoxycillin trihydrate and potassium clavulanate in combination, in a weight ratio of about 8:1 (the weights being expressed as the free parent acids amoxycillin and clavulanic acid).

2. A pharmaceutical formulation as claimed in claim 1 which, when reconstituted, comprises amoxycillin in an amount of from 200 to 800mg/5ml of liquid aqueous suspension plus a pro rata amount of potassium clavulanate .

3. A formulation as claimed in claim 1 or 2 in which the proportion of amoxycillin and clavulanic acid is from 35-60 wt%, in a dry formulation for make up with aqueous media into a suspension formulation.

4. A formulation as claimed in any one of claims 1 to 3 which is substantially free of mannitol.

5. A process for manufacturing a formulation according to any one of claims 1 to 4 comprising the step of mixing dry powdered or granulated ingredients for loading into a suitable container.

6. A formulation as claimed in claim 1 for use in therapy.
